(19) Europäisches Patentamt
European Patent Office
Office européen des brevets

(11) Veröffentlichungsnummer: **0 126 358**
**A2**

(12) # EUROPÄISCHE PATENTANMELDUNG

(21) Anmeldenummer: **84105085.9**

(22) Anmeldetag: **05.05.84**

(51) Int. Cl.³: **C 07 C 69/712**, C 07 C 67/14, C 07 D 213/64, C 07 C 67/31, C 07 C 67/11, A 01 N 31/02, A 01 N 43/40

(30) Priorität: **20.05.83 DE 3318353**

(43) Veröffentlichungstag der Anmeldung: **28.11.84**
**Patentblatt 84/48**

(84) Benannte Vertragsstaaten: **AT BE CH DE FR GB IT LI NL**

(71) Anmelder: **BAYER AG, Konzernverwaltung RP**
**Patentabteilung, D-5090 Leverkusen 1 Bayerwerk (DE)**

(72) Erfinder: **Förster, Heinz, Dr., Am Eckbusch 47, D-5600 Wuppertal 1 (DE)**
Erfinder: **Gallenkamp, Bernd, Dr., Claudiusweg 5, D-5600 Wuppertal 1 (DE)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**
Erfinder: **Eue, Ludwig, Dr., Paul-Klee-Strasse 36, D-5090 Leverkusen 1 (DE)**
Erfinder: **Schmidt, Robert R., Dr., Im Waldwinkel 110, D-5060 Bergisch-Gladbach 2 (DE)**

(54) **Optisch aktive Propionsäure-ester-Derivate.**

(57) Neue R-Enantiomere von Propionsäure-ester-Derivaten der Formel

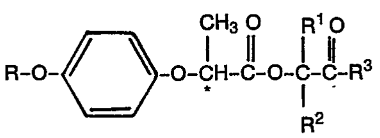

(I)

in welcher

R, $R^1$, $R^2$ und $R^3$ die in der Beschreibung angegebene Bedeutung haben,
mehrere Verfahren zur Herstellung dieser neuen Stoffe und deren Verwendung als Herbizide.

EP 0 126 358 A2

BAYER AKTIENGESELLSCHAFT 5090 Leverkusen, Bayerwerk

Konzernverwaltung RP
Patentabteilung

Dü/m-c
Ib

# Optisch aktive Propionsäure-ester-Derivate

Die Erfindung betrifft neue R-Enantiomere*) von Propionsäure-ester-Derivaten, mehrere Verfahren zu deren Herstellung und ihre Verwendung als Herbizide.

Es ist bereits bekannt geworden, daß zahlreiche Phenoxypropionsäure-Derivate herbizide Eigenschaften besitzen (vgl. DE-OS 26 28 384 und DE-OS 28 12 571). So können zum Beispiel das Racemat des 2-[4-(4-Trifluormethyl-phenoxy)-phenoxy]-propionsäure-(ethoxycarbonyl)-methylesters und das Racemat des 2-[4-(5-Trifluormethyl-pyridyl-2-oxy)-phenoxy]-propionsäure-(ethoxycarbonyl)-methylesters zur Unkrautbekämpfung eingesetzt werden. Die Wirkung dieser Stoffe ist gut, läßt jedoch bei niedrigen Aufwandmengen zu wünschen übrig.

---

*) Unter R-Enantiomeren sind hier jeweils diejenigen optisch aktiven Verbindungen zu verstehen, die am asymmetrischen Kohlenstoffatom der Propionsäure-Einheit die R-Konfiguration aufweisen.

Es wurden nun neue R-Enantiomere von Propionsäure-ester-Derivaten der Formel

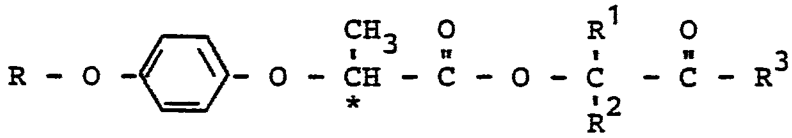 (I)

in welcher

R für die Reste der Formeln

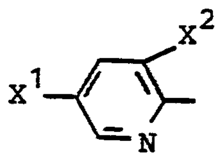 oder 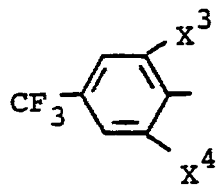 steht,

worin

$X^1$ für Halogen oder Trifluormethyl steht,

$X^2$ für Wasserstoff, Halogen oder Trifluormethyl steht,

$X^3$ für Wasserstoff oder Halogen steht und

$X^4$ für Wasserstoff oder Halogen steht,

$R^1$ für Wasserstoff, Alkyl mit 1 bis 4 Kohlenstoffatomen oder Halogenalkyl mit 1 bis 4 Kohlenstoffatomen und 1 bis 5 Halogenatomen steht,

$R^2$ für Wasserstoff oder Alkyl mit 1 bis 4 Kohlenstoffatomen steht und

$R^3$ für Alkoxy, Alkylthio oder den Rest 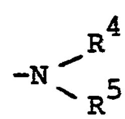 steht,

worin

$R^4$ und $R^5$ unabhängig voneinander für Wasserstoff, Alkyl oder Aryl stehen oder

$R^4$ und $R^5$ gemeinsam mit dem Stickstoffatom, an das sie gebunden sind, für einen gesättigten oder ungesättigten, 5- oder 6-gliedrigen heterocyclischen Ring stehen,

gefunden.

Weiterhin wurde gefunden, daß man die neuen R-Enantiomeren der Propionsäure-ester-Derivate der Formel (I) erhält, wenn man

a) R-Enantiomere von Phenoxypropionsäurechloriden der Formel

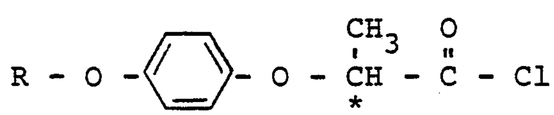

(II)

in welcher

R die oben angegebene Bedeutung hat,

mit Hydroxycarbonsäure-Derivaten der Formel

$$HO - \overset{R^1}{\underset{R^2}{C}} - \overset{O}{\overset{\|}{C}} - R^3 \qquad (III)$$

in welcher

$R^1$, $R^2$ und $R^3$ die oben angegebene Bedeutung haben,

gegebenenfalls in Gegenwart eines Säureakzeptors sowie gegebenenfalls in Gegenwart eines Verdünnungsmittles umsetzt,

oder

b) Phenol-Derivate der Formel

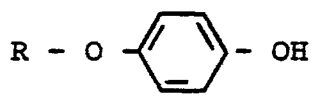

(IV)

in welcher

R die oben angegebene Bedeutung hat,

mit S-Enantiomeren von Propionsäure-Derivaten der Formel

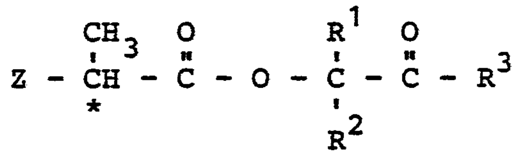

(V)

in welcher

$R^1$, $R^2$ und $R^3$ die oben angegebene Bedeutung haben und

Z für Chlor, Brom, Tosylat oder Mesylat steht,

gegebenenfalls in Gegenwart eines Säureakzeptors sowie gegebenenfalls in Gegenwart eines Verdünnungsmittels umsetzt,

oder

c) R-Enantiomere von Phenoxypropionsäure-Derivaten der Formel

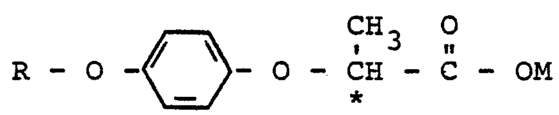

(VI)

in welcher

R die oben angegebene Bedeutung hat und

M für ein Alkalimetall-Kation oder für ein Äquivalent eines Erdalkalimetall-Kations steht,

mit Halogenverbindungen der Formel

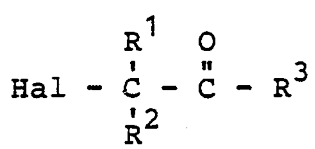

(VII)

in welcher

$R^1$, $R^2$ und $R^3$ die oben angegebene Bedeutung haben und

Hal für Chlor oder Brom steht,

gegebenenfalls in Gegenwart eines Säureakzeptors sowie gegebenenfalls in Gegenwart eines Verdünnungsmittels umsetzt.

Schließlich wurde gefunden, daß sich die neuen R-Enantiomeren von Propionsäure-ester-Derivaten der Formel (I) durch eine hervorragende herbizide Wirksamkeit auszeichnen.

Überraschenderweise besitzen die erfindungsgemäßen R-Enantiomeren der Propionsäure-ester-Derivate der Formel (I) wesentlich bessere herbizide Eigenschaften als die aus dem Stand der Technik bekannten Racemate des 2-[4-(4-Trifluormethyl-phenoxy)-phenoxy]-propionsäure-(ethoxycarbonyl)-methylesters und des 2-[4-(5-Trifluormethyl-pyridyl-2-oxy)-phenoxy]-propionsäure-(ethoxycarbonyl)-methylesters, welches die konstitutionell ähnlichsten vorbekannten Wirkstoffe analoger Wirkungsart sind.

Die erfindungsgemäßen Verbindungen sind durch die Formel (I) allgemein definiert. In dieser Formel, in der das asymmetrische Kohlenstoffatom durch ein (*) gekennzeichnet ist, steht R für die Reste der Formel

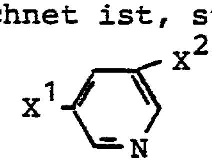 oder 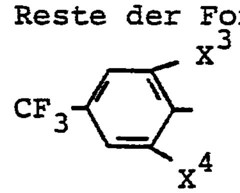 ,

in welchen

$X^1$ vorzugsweise für Chlor oder Trifluormethyl steht und
$X^2$ vorzugsweise für Wasserstoff oder Chlor steht, wobei

$X^2$ insbesondere dann für Wasserstoff steht, wenn $X^1$ für Trifluormethyl steht, und $X^2$ insbesondere dann für Chlor steht, wenn auch $X^1$ für Chlor steht, und in welchen ferner $X^3$ vorzugsweise für Wasserstoff oder Chlor steht und $X^4$ vorzugsweise für Wasserstoff oder Chlor steht.

$R^1$ steht vorzugsweise für Wasserstoff, Methyl, Ethyl oder Halogenalkyl mit 1 oder 2 Kohlenstoffatomen und 1 bis 3 Halogenatomen, insbesondere Fluor- und/oder Chloratomen. $R^2$ steht vorzugsweise für Wasserstoff oder Methyl, und $R^3$ steht vorzugsweise für Alkoxy mit 1 bis 4 Kohlenstoffatomen, Alkylthio mit 1 bis 4 Kohlenstoffatomen oder den Rest $-N{<}^{R^4}_{R^5}$, in welchem $R^4$ und $R^5$ unabhängig voneinander vorzugsweise für Wasserstoff, Alkyl mit 1 bis 4 Kohlenstoffatomen oder Phenyl stehen, und $R^4$ und $R^5$ außerdem gemeinsam mit dem Stickstoffatom, an das sie gebunden sind, vorzugsweise für Pyrrolyl, Pyridyl, Pyrrolidyl, Piperidyl, Morpholinyl, Piperazinyl, Pyrazolyl, Imidazolyl, 1,2,4-Triazol-1-yl-1,2,3-Triazol-1-yl oder 1,3,4-Triazol-1-yl stehen.

Diejenigen R-Enantiomeren der Verbindungen der Formel (I), in denen $R^1$ und $R^2$ verschieden sind, enthalten ein zweites Asymmetriezentrum in der Ester-Einheit. Die betreffenden Stoffe können daher in den folgenden Formen vorliegen:

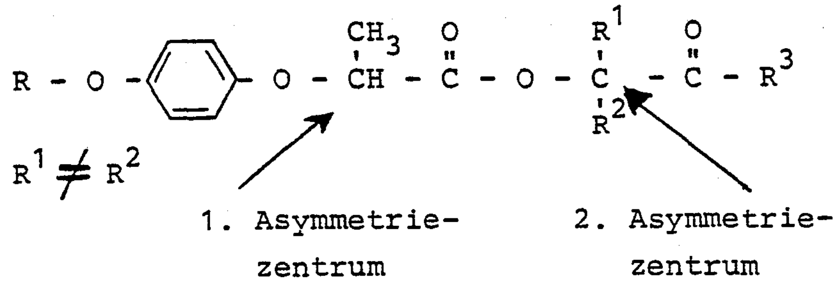


(I)

$R^1 \neq R^2$

| Konfiguration am 1. Asymmetriezentrum | Konfiguration am 2. Asymmetriezentrum |
|---|---|
| R | R |
| R | S |
| R | R/S (Racemat) |

Die vorliegende Erfindung umfaßt sowohl diejenigen Verbindungen, die am zweiten Asymmetriezentrum die R- oder die S-Konfiguration aufweisen, als auch die entsprechenden Racemate.

Verwendet man das R-Enantiomere des 2-/4-(4-Trifluormethyl-phenoxy)-phenoxy/-propionsäurechlorids und Hydroxyessigsäure-ethylester als Ausgangsstoffe, so kann der Verlauf des erfindungsgemäßen Verfahrens (a) durch das folgende Formelschema wiedergegeben werden:

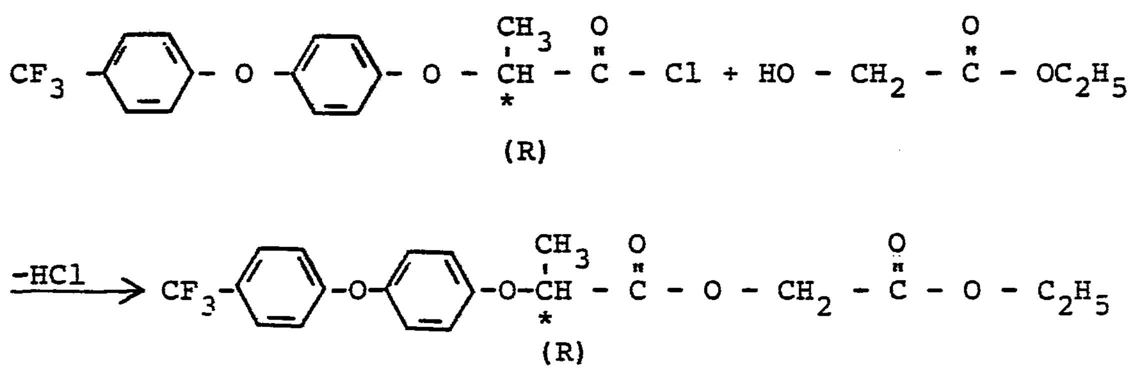


Verwendet man 4-(2-Chlor-4-trifluormethyl-phenoxy)-phenol und das S-Enantiomere des 2-Tosyloxy-propionsäure-(ethoxycarbonyl)-methylesters als Ausgangsstoffe,

so kann der Verlauf des erfindungsgemäßen Verfahrens (b) durch das folgende Formelschema wiedergegeben werden:

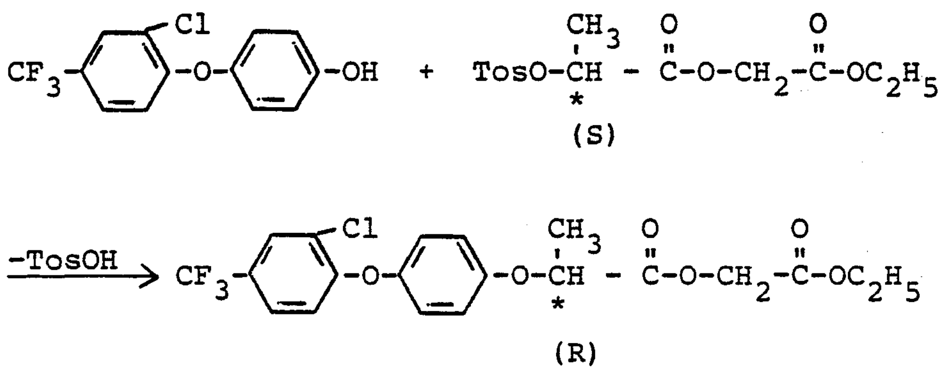


(S)

(R)

Tos = 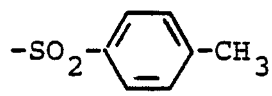 (= Tosyl)

Verwendet man das R-Enantiomere des 2-[4-(5-Trifluormethyl-pyridyl-2-oxy)-phenoxy]-propionsäure-Natriumsalzes und Bromessigsäure-ethylester als Ausgangsstoffe, so kann der Verlauf des erfindungsgemäßen Verfahrens (c) durch das folgende Formelschema wiedergegeben werden:

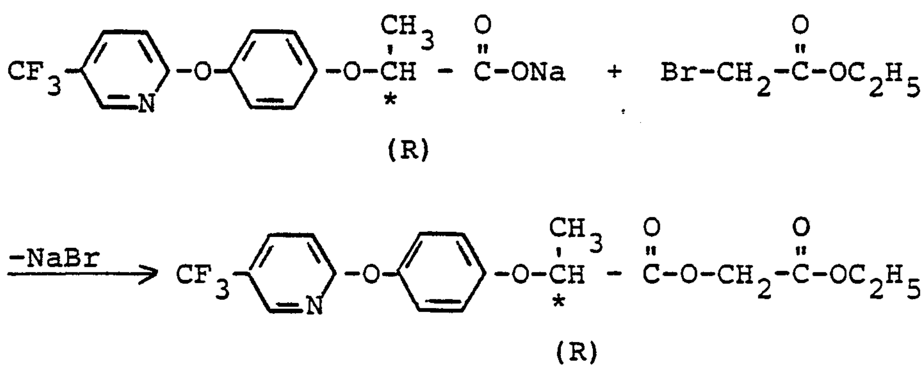


(R)

(R)

Die bei dem erfindungsgemäßen Verfahren (a) als Ausgangsstoffe benötigten R-Enantiomeren von Phenoxypropionsäurechloriden sind durch die Formel (II) allgemein definiert. In dieser Formel steht R vorzugsweise für diejenigen Reste, die bereits im Zusammenhang mit der Beschreibung der erfindungsgemäßen Stoffe vorzugsweise für den Substituenten R genannt wurden.

Die R-Enantiomeren der Phenoxypropionsäurechloride der Formel (II) sind bekannt oder lassen sich nach bekannten Methoden in einfacher Weise herstellen (vgl. DE-OS 2 758 002). So kann man die Stoffe der Formel (II) zum Beispiel dadurch erhalten, daß man die zugrundeliegenden Säuren mit Thionylchlorid umsetzt.

Die bei dem erfindungsgemäßen Verfahren (a) weiterhin als Ausgangsstoffe benötigten Hydroxycarbonsäure-Derivate sind durch die Formel (III) allgemein definiert. In dieser Formel haben $R^1$, $R^2$ und $R^3$ vorzugsweise diejenigen Bedeutungen, die bereits im Zusammenhang mit der Beschreibung der erfindungsgemäßen Stoffe der Formel (I) für diese Reste genannt wurden.

Die Hydroxycarbonsäure-Derivate der Formel (III) sind bekannt oder lassen sich nach bekannten Methoden in einfacher Weise herstellen (vgl. DE-OS 29 06 087).

Diejenigen Stoffe der Formel (III), in denen $R^1$ und $R^2$ verschieden sind, können entweder als Racemate oder als R- bzw. S-Enantiomere vorliegen. Verwendet man bei dem

erfindungsgemäßen Verfahren (a) die R-Enantiomeren der Hydroxycarbonsäure-Derivate der Formel (III), so entstehen diejenigen erfindungsgemäßen Stoffe, die am zweiten Asymmetriezentrum (vgl. oben) die R-Konfiguration aufweisen. Entsprechend entstehen aus den S-Enantiomeren diejenigen erfindungsgemäßen Stoffe, die am zweiten Asymmetriezentrum S-Konfiguration besitzen. Bei Verwendung von Racematen der Hydroxycarbonsäure-Derivate der Formel (III) fallen erfindungsgemäße Stoffe an, in denen das asymmetrisch substituierte Kohlenstoffatom im Ester-Teil keinen Beitrag zur optischen Aktivität leistet.

Auch die optisch aktiven Hydroxycarbonsäure-Derivate der Formel (III) sind bekannt oder lassen sich nach bekannten Methoden der Racemat-Spaltung herstellen.

Die bei dem erfindungsgemäßen Verfahren (b) als Ausgangsstoffe benötigten Phenol-Derivate sind durch die Formel (IV) definiert. In dieser Formel hat R vorzugsweise diejenigen Bedeutungen, die bereits im Zusammenhang mit der Beschreibung der erfindungsgemäßen Stoffe der Formel (I) vorzugsweise für diesen Substituenten genannt wurden.

Die Phenol-Derivate der Formel (IV) sind bekannt oder lassen sich nach bekannten Methoden in einfacher Weise herstellen (vgl. DE-OS 2 758 002, DE-OS 2 812 571, EP-OS 483 und EP-OS 1473).

Die bei dem erfindungsgemäßen Verfahren (b) weiterhin als Ausgangsstoffe benötigten S-Enantiomeren von Propionsäure-Derivaten sind durch die Formel (V) allgemein definiert. In dieser Formel haben $R^1$, $R^2$ und $R^3$ vorzugsweise diejenigen Bedeutungen, die bereits im Zusammenhang mit der Beschreibung der erfindungsgemäßen Stoffe der Formel (I) vorzugsweise für diese Reste genannt wurden. Z steht für Chlor, Brom, Tosylat ($-OSO_2-$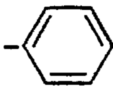$-CH_3$) oder Mesylat ($-OSO_2-CH_3$).

Diejenigen Stoffe der Formel (V), in denen $R^1$ und $R^2$ verschieden sind, können wiederum im Ester-Teil ein zweites Asymmetriezentrum enthalten, das in der R- oder in der S-Konfiguration vorliegen kann. In den entsprechenden Racematen leistet das asymmetrisch substituierte Kohlenstoffatom im Ester-Teil keinen Beitrag zur optischen Aktivität der S-Enantiomeren der Propionsäure-Derivate der Formel (V).

Die S-Enantiomeren der Propionsäure-Derivate der Formel (V) sind bekannt oder lassen sich nach üblichen Methoden in einfacher Weise herstellen. So erhält man die S-Enantiomeren der Propionsäure-Derivate der Formel (V), indem man S-Enantiomere von Propionylchloriden der Formel

$$Z - \underset{*}{\overset{CH_3}{\overset{|}{CH}}} - CO - Cl \qquad \text{(VIII)}$$

in welcher

Z die oben angegebene Bedeutung hat,

mit Hydroxycarbonsäure-Derivaten der Formel

$$HO - \overset{R^1}{\underset{R^2}{C}} - \overset{O}{\overset{\|}{C}} - R^3 \qquad (III)$$

in welcher

$R^1$, $R^2$ und $R^3$ die oben angegebene Bedeutung haben,

gegebenenfalls in Gegenwart eines Säureakzeptors sowie gegebenenfalls in Gegenwart eines Verdünnungsmittels umsetzt.

Bei diesem Verfahren zur Herstellung der S-Enantiomeren der Propionsäure-Derivate der Formel (V) entsprechen die Umsetzungsbedingungen denen des erfindungsgemäßen Verfahrens (a).

Der Einsatz von S-Enantiomeren der Phenoxypropionsäure-Derivate der Formel (V) ist bei dem erfindungsgemäßen Verfahren (b) deshalb erforderlich, weil im Verlauf der Reaktion eine Walden'sche Umkehr am asymmetrisch substituierten Kohlenstoffatom der Propionsäure-Einheit erfolgt.

Die bei dem erfindungsgemäßen Verfahren (c) als Ausgangsstoffe benötigten R-Enantiomeren der Phenoxypropionsäure-Derivate sind durch die Formel (VI) definiert. In dieser Formel hat R vorzugsweise diejenigen Bedeutungen, die bereits im Zusammenhang mit der Beschreibung der erfindungsgemäßen Stoffe der Formel (I) vorzugsweise für diesen Rest genannt wurden. M steht vorzugsweise für ein Natrium- oder Kalium-Kation, oder für ein Äquivalent eines Calcium-Kations.

Die R-Enantiomeren der Phenoxypropionsäure-Derivate der Formel (VI) sind bekannt oder lassen sich in einfacher Weise nach bekannten Verfahren herstellen (vgl. DE-OS 27 58 002).

Man erhält die R-Enantiomeren der Phenoxypropionsäure-Derivate der Formel (VI) zum Beispiel dadurch, daß man Phenol-Derivate der Formel

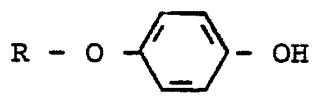 (IV)

in welcher

R die oben angegebene Bedeutung hat,

mit S-Enantiomeren der Milchsäure-ester der Formel

$$Q - \underset{*}{\overset{CH_3}{\overset{|}{CH}}} - COOR^6 \qquad (IX)$$

in welcher

$R^6$ für Methyl oder Ethyl steht und
Q für Tosylat oder Mesylat steht,

gegebenenfalls in Gegenwart eines Säureakzeptors, wie zum Beispiel Kaliumcarbonat, und gegebenenfalls in Gegenwart eines Verdünnungsmittels, wie zum Beispiel Acetonitril, bei Temperaturen zwischen 0°C und 120°C umsetzt und die dabei entstehenden R-Enantiomeren der Ester der Formel

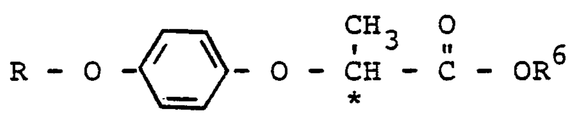

(X)

in welcher

R und $R^6$ die oben angegebene Bedeutung haben,

mit Basen der Formel

M OH (XI)

in welcher

M die oben angegebene Bedeutung hat,

in Gegenwart eines Verdünnungsmittels, wie zum Beispiel Methanol, Ethanol, Benzol, Toluol oder Xylol, gegebenenfalls im Gemisch mit Wasser bei Temperaturen zwischen 20°C und 140°C umsetzt.

In der ersten Stufe der obigen Umsetzung findet am asymmetrisch substituierten Kohlenstoffatom des jeweiligen Milchsäure-esters der Formel (IX) eine Walden'sche

Umkehr statt. Dieses hat zur Folge, daß durch Umsetzung der Phenol-Derivate der Formel (IV) mit den S-Enantiomeren der Milchsäure-ester der Formel (IX) die R-Enantiomeren der Ester der Formel (X) entstehen.

Die bei dem erfindungsgemäßen Verfahren (c) weiterhin als Ausgangsstoffe benötigten Halogenverbindungen sind durch die Formel (VII) definiert. In dieser Formel haben $R^1$, $R^2$ und $R^3$ vorzugsweise diejenigen Bedeutungen, die bereits im Zusammenhang mit der Beschreibung der erfindungsgemäßen Stoffe der Formel (I) vorzugsweise für diese Reste genannt wurden. Hal steht für Chlor oder Brom.

Die Halogenverbindungen der Formel (VII) sind bekannt oder lassen sich in einfacher Weise nach bekannten Methoden herstellen.

Diejenigen Stoffe der Formel (VII), in denen $R^1$ und $R^2$ verschieden sind, können entweder als Racemate oder als R- bzw. S-Enantiomere vorliegen. Verwendet man bei dem erfindungsgemäßen Verfahren (c) die R-Enantiomeren der Halogenverbindungen der Formel (VII), so entstehen diejenigen erfindungsgemäßen Stoffe, die am zweiten Asymmetriezentrum (vgl. oben) die S-Konfiguration aufweisen. Entsprechend entstehen aus den S-Enantiomeren diejenigen erfindungsgemäßen Stoffe, die am zweiten Asymmetriezentrum die R-Konfiguration besitzen. Bei Verwendung von Racematen der Halogenverbindungen der Formel (VII) fallen erfindungsgemäße Stoffe an, in denen das asymmetrisch substituierte Kohlenstoffatom im Ester-Teil keinen Beitrag zur optischen Aktivität leistet.

Die erfindungsgemäßen Verfahren (a), (b) und (c) zur Herstellung der neuen R-Enantiomeren der Propionsäureester-Derivate der Formel (I) werden vorzugsweise unter Verwendung von Verdünnungsmitteln durchgeführt. Als Verdünnungsmittel kommen praktisch alle inerten organischen Lösungsmittel in Frage. Hierzu gehören vorzugsweise aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methylisopropyl-, und Methyl-isobutyl-keton, Ester, wie Essigsäure-methylester und -ethylester, Nitrile, wie zum Beispiel Acetonitril und Propionitril, Amide, wie zum Beispiel Dimethylformamid, Dimethylacetamid und N-Methyl-pyrrolidon sowie Dimethylsulfoxid, Tetramethylsulfon und Hexamethylphosphorsäuretriamid.

Als Säurebindemittel können sowohl bei den erfindungsgemäßen Verfahren (a) und (b) als auch bei dem Verfahren (c) alle üblicherweise für derartige Umsetzungen verwendbaren Säurebindemittel eingesetzt werden. Vorzugsweise in Frage kommenden Alkalihydroxide, wie zum Beispiel Natrium- und Kaliumhydroxid, Alkalicarbonate und -alkoholate, wie Natrium- und Kaliumcarbonat, Natrium- und Kaliummethylat bzw. -ethylat, ferner aliphatische,

aromatische oder heterocyclische Amine, beispielsweise Triethylamin, Trimethylamin, Dimethylanilin, Dimethylbenzylamin, Pyridin, 1,5-Diaza-bicyclo-/4.3.0/-nonen-5 (DBN) und 1,8-Diaza-bicyclo-/5.4.0/-undec-7-en (DBU).

Die Reaktionstemperaturen können sowohl bei dem erfindungsgemäßen Verfahren (a) als auch bei den Verfahren (b) und (c) innerhalb eines größeren Bereiches variiert werden. Im allgemeinen arbeitet man jeweils bei Temperaturen zwischen -20°C und +160°C, vorzugsweise zwischen 0°C und 140°C.

Die erfindungsgemäßen Verfahren (a), (b) und (c) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a), (b) und (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a), (b) und (c) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen übergeführt werden, wie Lösungen, Emulsionen, Spritzpulver, Suspensionen, Pulver, Stäubemittel, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage:

Aromaten, wie Xylol, Toluol, oder Alkylnaphthaline, chlorierte Aromaten oder chlorierte aliphatische Kohlenwasserstoffe, wie Chlorbenzol, Chlorethylen oder Methylenchlorid, aliphatische Kohlenwasserstoffe, wie Cyclohexan oder Paraffine, z.B. Erdölfraktionen, Alkohole, wie Butanol oder Glycol sowie deren Ester, Ketone, wie Aceton, Methylethylketon, Methylisobutylketon oder Cyclohexanon, stark polare Lösungsmittel, wie Dimethylformamid und Dimethylsulfoxid, sowie Wasser und mineralische oder pflanzliche Öle.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate sowie Ammoniumsalze; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischemen Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azofarbstoffe, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist.

Für die Mischungen kommen bekannte Herbizide wie zum Beispiel 1-Amino-6-ethylthio-3-(2,2-dimethylpropyl)-1,3,5-triazin-2,4-(1H, 3H)-dion oder N-(2-Benzthiazolyl)-N,N'-dimethyl-harnstoff zur Unkrautbekämpfung in Getreide, 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen in Frage. Einige dieser Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver und Granulate angewandt werden. Die Anwendung geschieht in üblicher Weise z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,05 und 10 kg Wirkstoff pro ha, vorzugsweise zwischen 0,1 und 5 kg/ha.

Die Herstellung und die Verwendung der erfindungsgemäßen Stoffe geht aus den nachfolgenden Beispielen hervor.

## Herstellungsbeispiele

### Beispiel 1

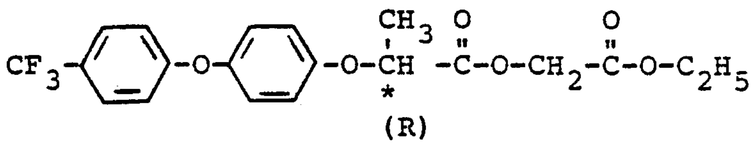

Verfahren a:

Eine Lösung von 8,6 g (0,025 Mol) des R-Enantiomeren des 2-/4-(4-Trifluormethyl-phenoxy)-phenoxy7-propionsäure-chlorids in 50 ml Toluol wurde bei Temperaturen zwischen 0°C und +5°C in ein Gemisch aus 2,6 g (0,025 Mol) Hydroxyessigsäure-ethylester und 2,8 g (0,0275 Mol) Triethylamin in 50 ml Toluol gegeben. Man rührte weitere 14 Stunden beim Raumtemperatur und arbeitete dann auf, indem man das Reaktionsgemisch zunächst mit Wasser versetzte, dann mit Toluol extrahierte, die vereinigten organischen Phasen nacheinander mit verdünnter wäßriger Salzsäure und Wasser wusch, dann trocknete und durch Abziehen des Lösungsmittels unter vermindertem Druck einengte. Der verbleibende Rückstand wurde durch leichtes Erwärmen im Hochvakuum von Resten an flüchtigen Anteilen befreit. Man erhielt auf diese Weise 7,7 g (74,8 % der Theorie) an dem R-Enantiomeren des 2-/4-(4-Trifluormethyl-phenoxy)-phenoxy7-propionsäure-(ethoxycarbonyl)-methyl-esters.

Brechungsindex: $n_D^{20,5} = 1,5029$

Drehwert: $[\alpha]_D^{24} = + 10,5°$
(1-molare Lösung in Chloroform; Küvettenlänge = 10 cm).

Verfahren b:

Ein Gemisch aus 12,7 g (0,05 Mol) 4-(4-Trifluormethyl-phenoxy)-phenol, 17,7 g (0,05 Mol) an S-Enantiomerem des 2-Tosyloxy-propionsäure-(ethoxycarbonyl)-methylesters und 8 g (0,057 Mol) Kaliumcarbonat in 100 ml Acetonitril wurde 6 Stunden unter Rückfluß erhitzt. Danach wurde das Reaktionsgemisch auf Raumtemperatur abgekühlt und mit 400 ml Wasser versetzt. Es wurde zweimal mit je 200 ml Toluol extrahiert. Die vereinigten organischen Phasen wurden über Natriumsulfat getrocknet und durch Abziehen des Lösungsmittels unter vermindertem Druck eingeengt. Der verbleibende Rückstand wurde durch leichtes Erwärmen im Hochvakuum von Resten an flüchtigen Anteilen befreit. Man erhielt auf diese Weise das R-Enantiomere des 2-[4-(4-Trifluormethyl.-phenoxy)-phenoxy]-propionsäure-(ethoxycarbonyl)-methylesters in einer Ausbeute von 53,5 % der Theorie.

Drehwert: $[\alpha]_D^{24} = + 9,6°$
(1-molare Lösung in Chloroform; Küvettenlänge = 10 cm)

Herstellung des Ausgangsproduktes der Formel

$$\text{TosO-}\underset{*}{\overset{CH_3}{\overset{|}{C}}}\text{H} - \overset{O}{\overset{\|}{C}}\text{-O-CH}_2\text{-COO-C}_2\text{H}_5 \qquad (V-1)$$

(S)

247 g (0,94 Mol) des S-Enantiomeren des Milchsäurechloridtosylates wurden bei 10°C unter Rühren in ein Gemisch aus 98 g (0,94 Mol) Hydroxyessigsäure-ethylester und 94 g (0,94 Mol) Triethylamin in 300 ml Toluol gegeben. Man rührte weitere 14 Stunden bei 20°C und arbeitete dann auf, indem man das Reaktionsgemisch mit 600 ml Wasser versetzte und die organische Phase abtrennte. Nach dem Abziehen des Lösungsmittels unter vermindertem Druck erhielt man auf diese Weise 285 g (93 % der Theorie) an dem S-Enantiomeren des 2-Tosyloxy-propionsäure-(ethoxycarbonyl)-methylesters.

Drehwert: $[\alpha]_D^{24}$ = -11,6°
(1-molare Lösung in Chloroform;
Küvettenlänge = 10 cm).

Nach den im Beispiel 1 angegebenen Methoden wurden auch die in den folgenden Beispielen formelmäßig aufgeführten Stoffe hergestellt.

Beispiel 2

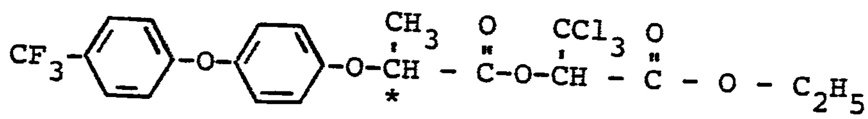

(R)

Ausbeute: 79,3 % der Theorie

Brechungsindex: $n_D^{20,5}$ = 1,5146

Drehwert: $[\alpha]_D^{24}$ = +16,0°
(1-molare Lösung in Chloroform;
Küvettenlänge = 10 cm)

Beispiel 3

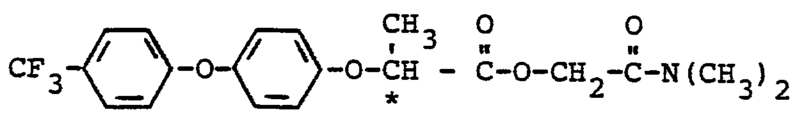

(R)

Ausbeute: 48,7 % der Theorie

Brechungsindex: $n_D^{20,5}$ = 1,5215

Drehwert: $[\alpha]_D^{24}$ = +12,2°
(1-molare Lösung in Chloroform;
Küvettenlänge = 10 cm)

Beispiel 4

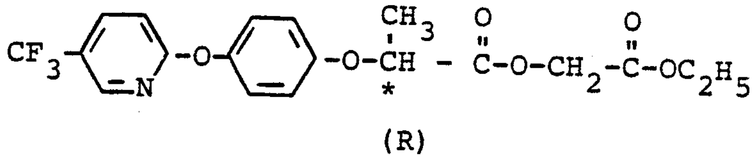

(R)

Ausbeute: 78,9 % der Theorie

Brechungsindex: $n_D^{21}$ = 1,5045

Drehwert: $[\alpha]_D^{24}$ = +15,7° (1-molare Lösung in Chloroform; Küvettenlänge = 10 cm).

Beispiel 5

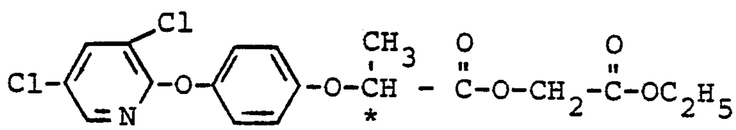

(R)

Ausbeute: 69 % der Theorie

Brechungsindex: $n_D^{20,5}$ = 1,5513

Drehwert: $[\alpha]_D^{24}$ = +13,2° (1-molare Lösung in Chloroform; Küvettenlänge = 10 cm)

Beispiel 6

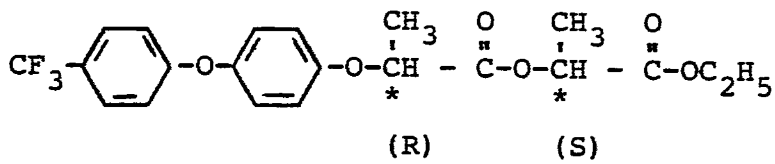

26 g (0,075 Mol) des zweifach S-konfigurierten Enantiomeren des 2-Tosyloxy-propionsäure-(1-ethoxycarbonyl)-ethylesters wurden in ein Gemisch aus 19 g (0,075 Mol) 4-(4-Trifluormethyl-phenoxy)-phenol und 12 g (0,87 Mol) Kaliumcarbonat in 220 ml Acetonitril gegeben. Das Gemisch wurde 15 Stunden unter Rückfluß erhitzt und anschließend abgekühlt auf Raumtemperatur. Danach wurde aufgearbeitet, indem man das Reaktionsgemisch mit Wasser versetzte, zweimal mit Toluol extrahierte, die vereinigten organischen Phasen mit Wasser wusch, trocknete und durch Abziehen des Lösungsmittels unter vermindertem Druck einengte. Man erhielt auf diese Weise 17 g (53 % der Theorie) an dem R/S konfigurierten Enantiomeren des 2-/4-(4-Trifluormethyl-phenoxy)-phenoxy7-propionsäure-(1-ethoxycarbonyl)-ethylesters der oben angegebenen Formel.

Drehwert: $[\alpha]_D^{24}$ = -2,5°.

Herstellung des Ausgangsproduktes der Formel

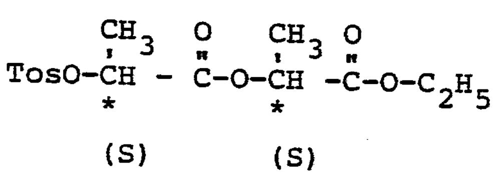

(V-2)

26,2 g (0,1 Mol) des S-Enantiomeren des Milchsäurechloridtosylates wurden bei 20°C unter Rühren in ein Gemisch aus 11,8 g (0,1 Mol) des S-Enantiomeren des Milchsäure-ethylesters und 10 g (0,1 Mol) Triethylamin in 150 ml Toluol gegeben. Das Reaktionsgemisch wurde weitere 14 Stunden bei Raumtemperatur gerührt und dann in Wasser gegossen. Die organische Phase wurde abgetrennt, mit Wasser gewaschen, getrocknet und durch Abziehen des Lösungsmittels eingeengt. Man erhielt auf diese Weise 27 g (76 % der Theorie) des zweifach S-konfigurierten Enantiomeren des 2-Tosyloxy-propionsäure-(1-ethoxycarbonyl)-ethylesters.
Drehwert: $[\alpha]_D^{24}$ = -17,8°.

In analoger Weise lassen sich die in den folgenden Beispielen formelmäßig aufgeführten Stoffe herstellen:

Beispiel 7

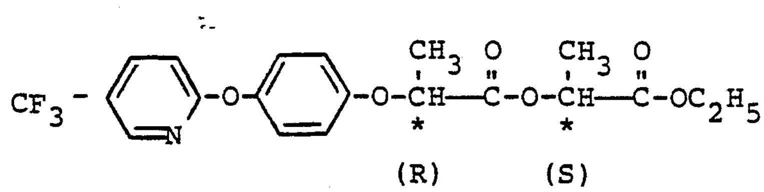

Ausbeute: 70,9 % der Theorie
Brechungsindex: $n_D^{21}$ : 1,5006
Drehwert: $[\alpha]_D^{24}$ = + 8,76°.

Beispiel 8

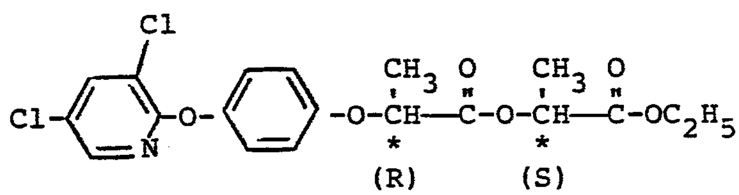

Ausbeute: 66,8 % der Theorie

Brechungsindex: $n_D^{20,5}$ = 1,5481

Drehwert: $[\alpha]_D^{24}$ = + 7,1°

Beispiel 9

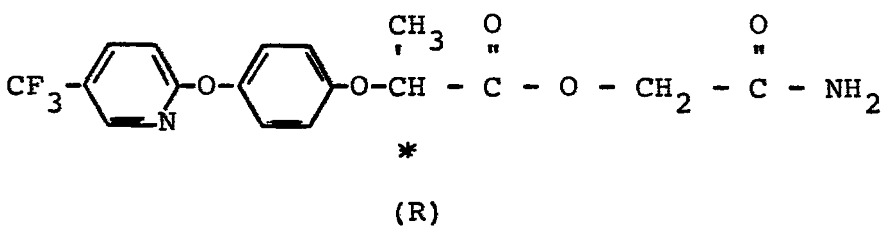

Brechungsindex: $n_D^{21}$ = 1,5259

Drehwert : $[\alpha]_D^{24}$ = + 7,77°

Beispiel 1o

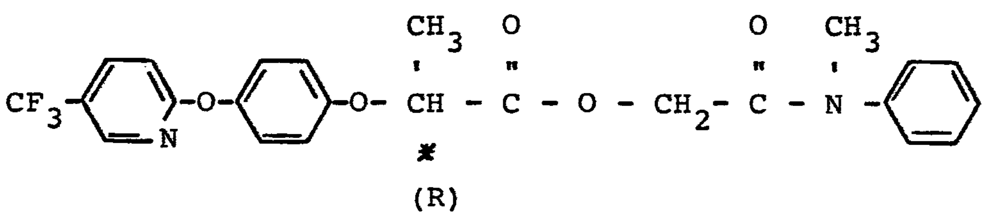

Brechungsindex: $n_D^{21}$ = 1,5415

Drehwert : $[\alpha]_D^{24}$ = + 23,46°

Beispiel 11

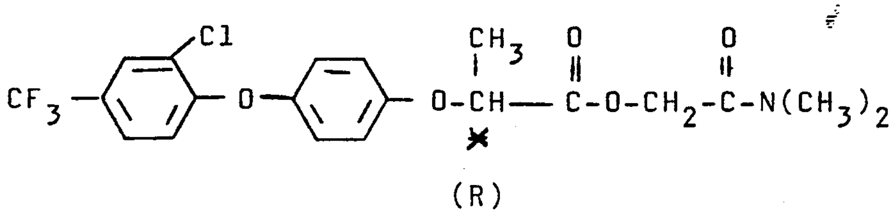

Beispiel 12

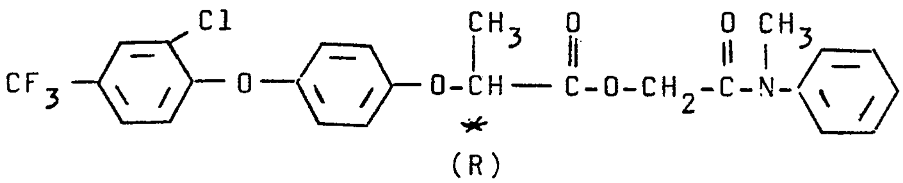

Beispiel 13

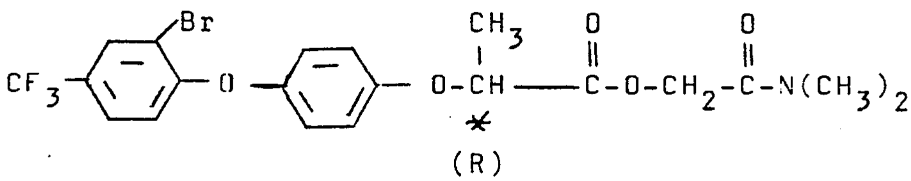

Beispiel 14

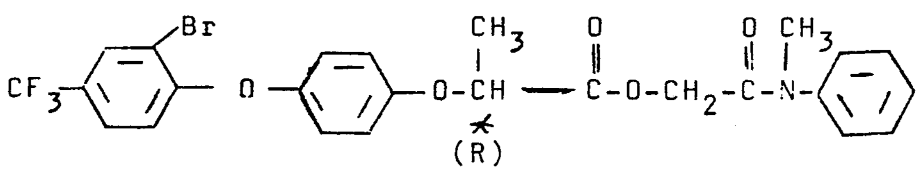

Beispiel 15

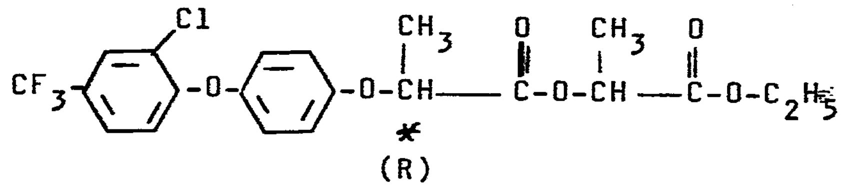

Beispiel 16

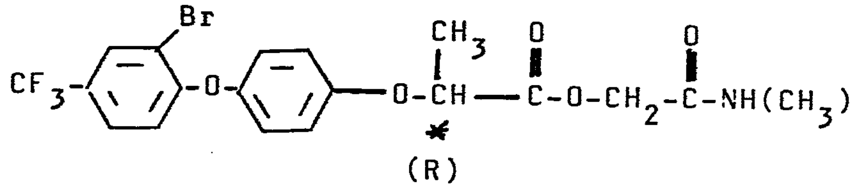

Beispiel 17

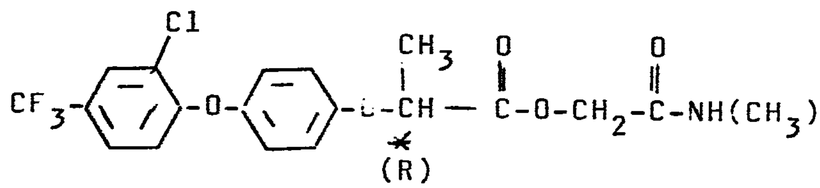

Beispiel 18

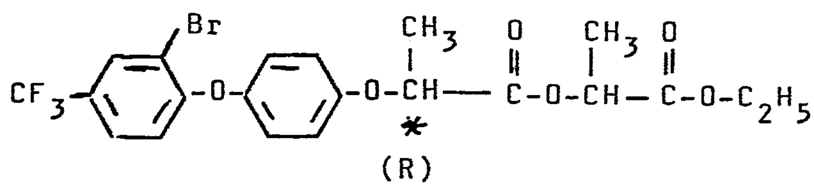

Beispiel 19

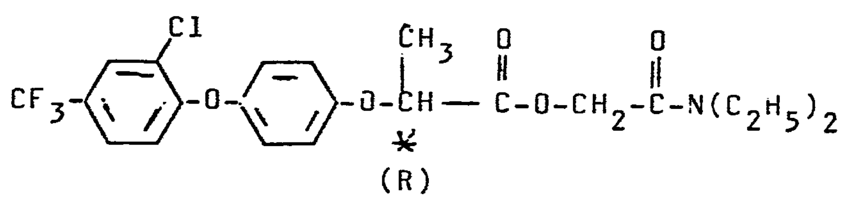

Beispiel 20

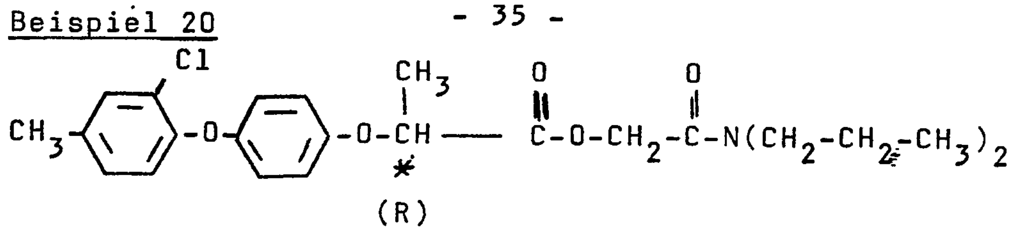

Beispiel 21

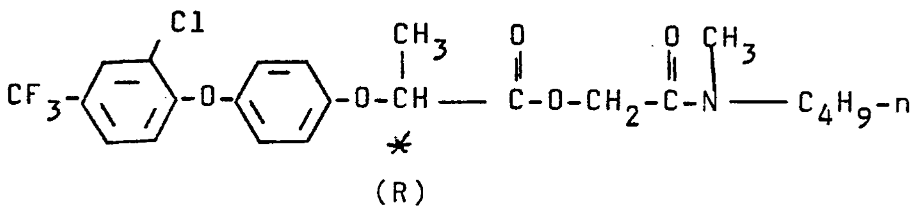

Beispiel 22

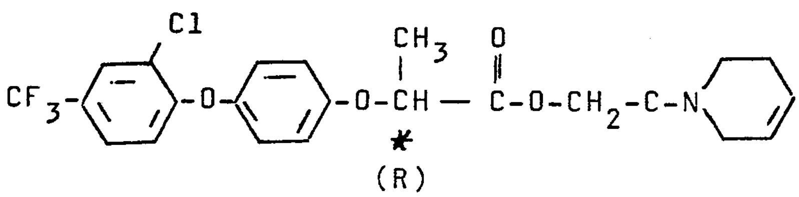

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Hohe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % keine Wirkung (wie unbehandelte Kontrolle)
100 % totale Vernichtung

Der Wirkstoff gemäß Beispiel 8 zeigt in diesem Test eine sehr gute Wirksamkeit bei der selektiven Bekämpfung von Panicum und Avena fatua in Rüben und Baumwolle.

Patentansprüche

1. R-Enantiomere von Propionsäure-ester Derivaten der Formel

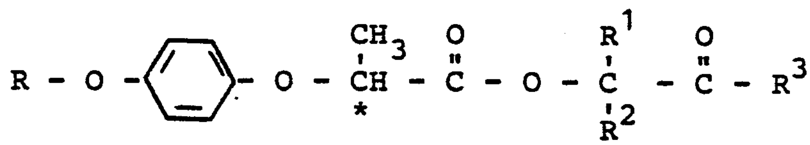

(I)

in welcher

R für die Reste der Formeln

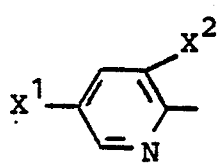

oder

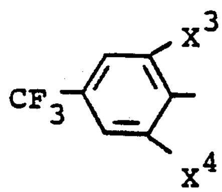

steht,

worin

$X^1$ für Halogen oder Trifluormethyl steht,

$X^2$ für Wasserstoff, Halogen oder Trifluormethyl steht und

$X^3$ für Wasserstoff oder Halogen steht und

$X^4$ für Wasserstoff oder Halogen steht,

$R^1$ für Wasserstoff, Alkyl mit 1 bis 4 Kohlenstoffatomen oder Halogenalkyl mit 1 bis 4 Kohlenstoffatomen und 1 bis 5 Halogenatomen steht,

$R^2$ für Wasserstoff oder Alkyl mit 1 bis 4 Kohlenstoffatomen steht und

$R^3$ für Alkoxy, Alkylthio oder den Rest

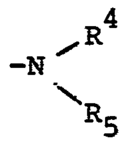

steht, worin

$R^4$ und $R^5$ unabhängig voneinander für Wasserstoff, Alkyl oder Aryl stehen oder

$R^4$ und $R^5$ gemeinsam mit dem Stickstoffatom, an das sie gebunden sind, für einen gesättigten oder ungesättigten, 5- oder 6-gliedrigen heterocyclischen Ring stehen.

2. R-Enantiomere von Propionsäure-ester-Derivaten der Formel (I), in denen

R für die Reste der Formeln

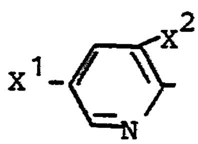

oder

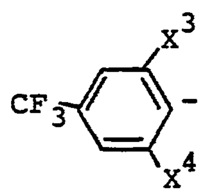

steht, worin

$X^1$ für Chlor oder Trifluormethyl steht,

$X^2$ für Wasserstoff oder Chlor steht,

$X^3$ für Wasserstoff oder Chlor steht und

$X^4$ für Wasserstoff oder Chlor steht,

$R^1$ für Wasserstoff, Methyl, Ethyl oder Halogenalkyl mit 1 oder 2 Kohlenstoffatomen und 1 bis 3 Halogenatomen steht,

$R^2$ für Wasserstoff oder Methyl steht, und

$R^3$ für Alkoxy mit 1 bis 4 Kohlenstoffatomen, Alkylthio mit 1 bis 4 Kohlenstoffatomen oder den Rest

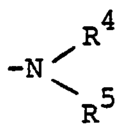 steht, in welchen

$R^4$ und $R^5$ unabhängig voneinander für Wasserstoff, Alkyl mit 1 bis 4 Kohlenstoffatomen oder Phenyl stehen,
oder

$R^4$ und $R^5$ gemeinsam mit dem Stickstoffatom, an das sie gebunden sind, für Pyrrolyl, Pyridyl, Pyrrolidyl, Piperidyl, Morpholinyl, Piperazinyl, Pyrazolyl, Imidazolyl, 1,2,4-Triazol-1-yl, 1,2,3-Triazol-1-yl oder 1,3,4-Triazol-1-yl stehen.

3. Verfahren zur Herstellung von R-Enantiomeren von Propionsäure-ester-Derivaten der Formel

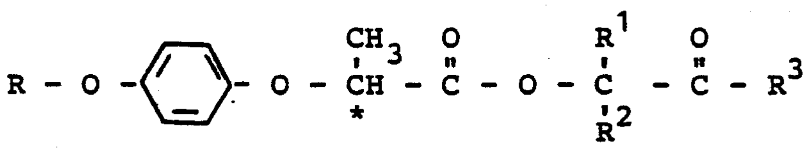

(I)

in welcher

R für die Reste der Formeln

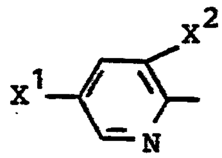 oder 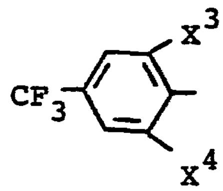 steht,

worin

$X^1$ für Halogen oder Trifluormethyl steht,

$X^2$ für Wasserstoff, Halogen oder Trifluormethyl steht,

$X^3$ für Wasserstoff oder Halogen steht und

$X^4$ für Wasserstoff oder Halogen steht,

$R^1$ für Wasserstoff, Alkyl mit 1 bis 4 Kohlenstoffstomen oder Halogenalkyl mit 1 bis 4 Kohlenstoffatomen und 1 bis 5 Halogenatomen steht,

$R^2$ für Wasserstoff oder Alkyl mit 1 bis 4 Kohlenstoffatomen steht und

$R^3$ für Alkoxy, Alkylthio oder den Rest

$$-N\begin{matrix} R^4 \\ R^5 \end{matrix}$$ steht, worin

$R^4$ und $R^5$ unabhängig voneinander für Wasserstoff, Alkyl oder Aryl stehen, oder

$R^4$ und $R^5$ gemeinsam mit dem Stickstoffatom, an das sie gebunden sind, für einen gesättigten oder ungesättigten, 5- oder 6-gliedrigen heterocyclischen Ring stehen,

dadurch gekennzeichnet, daß man

a) R-Enantiomere von Phenoxypropionsäurechloriden der Formel

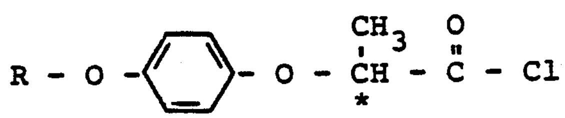

(II)

in welcher

R die oben angegebene Bedeutung hat,

mit Hydroxycarbonsäure-Derivaten der Formel

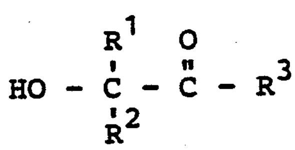

(III)

in welcher

$R^1$, $R^2$ und $R^3$ die oben angegebene Bedeutung haben,

gegebenenfalls in Gegenwart eines Säureakzeptors sowie gegebenenfalls in Gegenwart eines Verdünnungsmittels umsetzt,

oder

b) Phenol-Derivate der Formel

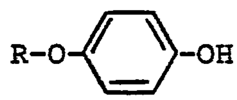

(IV)

in welcher

R die oben angegebene Bedeutung hat,

mit S-Enantiomeren von Propionsäure-Derivaten der Formel

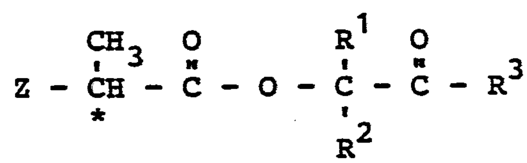

(V)

in welcher

$R^1$, $R^2$ und $R^3$ die oben angegebene Bedeutung haben und

Z für Chlor, Brom, Tosylat oder Mesylat steht,

gegebenenfalls in Gegenwart eines Säureakzeptors sowie gegebenenfalls in Gegenwart eines Verdünnungsmittels umsetzt, oder

c) R-Enantiomere von Phenoxypropionsäure-Derivaten der Formel

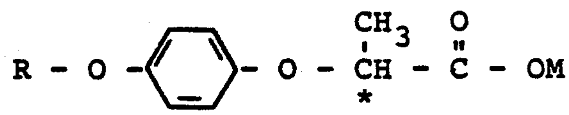

(VI)

in welcher

R die oben angegebene Bedeutung hat und

M für ein Alkalimetall-Kation oder für ein Äquivalent eines Erdalkalimetall-Kations steht,

mit Halogenverbindungen der Formel

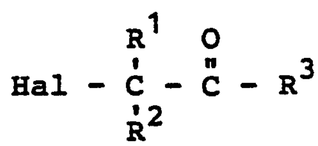

(VII)

in welcher

$R^1$, $R^2$ und $R^3$ die oben angegebene Bedeutung haben und

Hal für Chlor oder Brom steht,

gegebenenfalls in Gegenwart eines Säureakzeptors sowie gegebenenfalls in Gegenwart eines Verdünnungsmittels umsetzt.

4. Herbizide Mittel, gekennzeichnet durch einen Gehalt an mindestens einem R-Enantiomeren eines Propionsäure-ester-Derivates der Formel (I).

5. Verwendung von R-Enantiomeren von Propionsäure-ester-Derivaten der Formel (I) als Herbizide.

6. Verfahren zur Bekämpfung von Unkräutern, dadurch gekennzeichnet, daß man R-Enantiomere von Propionsäure-ester-Derivaten der Formel (I) auf die Unkräuter und/oder deren Lebensraum ausbringt.

7. Verfahren zur Herstellung von herbiziden Mitteln, dadurch gekennzeichnet, daß man R-Enantiomere von Propionsäure-ester-Derivaten der Formel (I) mit Streckmitteln und/oder oberflächenaktiven Stoffen vermischt.

8. Verbindung der Formel

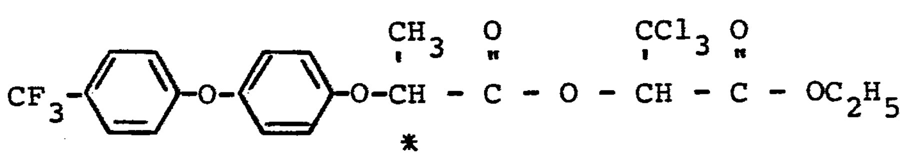

(R)

9. Verbindung der Formel

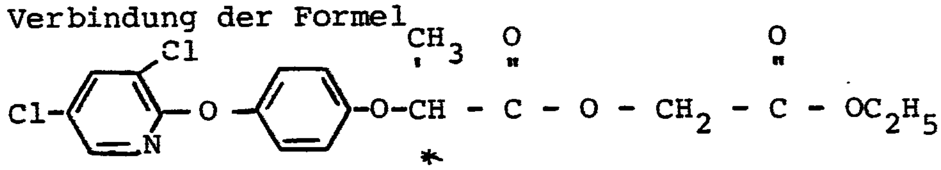

(R)

1o. Verbindung für Formel